# EUROPÄISCHE PATENTSCHRIFT

(11) **EP 1 778 690 B1**
(45) Veröffentlichungstag und Bekanntmachung des Hinweises auf die Patenterteilung: **22.08.2012**
(21) Anmeldenummer: 05770568.3
(22) Anmeldetag: 30.07.2005
(51) Int. Cl.: C07D 473/08, A61K 31/522, A61P 3/10

(54) **Substituierte 8-Aminoalkoxixanthine, Verfahren zu ihrer Herstellung und ihre Verwendung als Arzneimittel**
Substituted 8-aminoalkoxi-xanthines, method for the production thereof and use thereof as medicaments
8-Aminoalkoxixanthines substituées, leur procédé de preparation, et leur utilisation comme médicaments

(30) Priorität: 14.08.2004 DE 102004039507
(43) Veröffentlichungstag der Anmeldung: 02.05.2007
(73) Patentinhaber: Sanofi-Aventis Deutschland GmbH, 65929 Frankfurt am Main (DE)
(72) Erfinder: SCHOENAFINGER, Karl, 63755 Alzenau (DE); JAEHNE, Gerhard, 65929 Frankfurt (DE); DEFOSSA, Elisabeth, 65510 Idstein (DE); BUNING, Christian, 53175 Bonn (DE); TSCHANK, Georg, 55270 Essenheim (DE); WERNER, Ulrich, 56357 Miehlen (DE)
(86) Internationale Anmeldenummer: PCT/EP2005/008283
(87) Internationale Veröffentlichungsnummer: WO 2006/018117

(56) Entgegenhaltungen:
- EP-A- 0 430 300
- WO-A-92/05175
- US-A- 2 688 618
- DATABASE CAPLUS [Online] CHEMICAL ABSTRACTS SERVICE, COLUMBUS, OHIO, US; 2001, XP002361403 gefunden im STN Database accession no. 2001:714509 & E. TRUTA ET AL.: "The effects induced by 8-(4-R)-phenoxymethylxanthinic compounds on germination, plantlet growth and mitosis in Allium capa L." ANALELE STIINTIFICE ALE UNIVERSITATII AL. I. CUZA DIN IASI, SECTIUNEA 2A: GENETICA SI BIOLOGIE MOLECULARA, Bd. 2001, Nr. 1, 2000, Seiten 43-49, UNIVERSITATEA AL. I. CUZA: CARTIMEX, IASI; RO
- DATABASE CAPLUS [Online] CHEMICAL ABSTRACTS SERVICE, COLUMBUS, OHIO, US; 2001, XP002361404 gefunden im STN Database accession no. 1966:104502 & J. KEJHA ET AL.: "Iodinated phenoxyalkyltheophyllines" CESKA A SLOVENSKA FARMACIE, Bd. 15, Nr. 1, 1966, Seiten 2-4, CESKA LEKARSKA SPOLECNOST J.E. PURKYNE, PRAGUE; CZ
- DATABASE WPI Section Ch, Week 200443 Derwent Publications Ltd., London, GB; Class B02, AN 2004-460731 XP002361443 -& WO 2004/048379 A1 (SUMITOMO PHARM CO LTD) 10. Juni 2004 (2004-06-10)

## Beschreibung

Die Erfindung betrifft substituierte 8-Aminoalkoxi-xanthine sowie deren physiologisch verträgliche Salze und physiologisch funktionelle Derivate.

Strukturähnliche basische substituierte Coffeinderivate sind in US 2,688,618 beschrieben. WO92/05175 beschreibt strukturähnliche Verbindungen zur Behandlung von Allergien. XPO02361403 in STN Database acession no 2001:714509 und XP002361404 in STN Database acession no 1966:104502 beschreiben strukturähnliche Verbindungen. EP0430300 beschreibt strukturähnliche Verbindungen als Angiotensin II Inhibitoren. Derwent Week: 43, 2004, XPO023614443 in Internal Database acession no 2004-460731 beschreiben strukturähnliche Verbindungen. WO2004/048379 beschreibt strukturähnliche Verbindungen.

Der Erfindung lag die Aufgabe zugrunde, Verbindungen zur Verfügung zu stellen, die eine therapeutisch verwertbare Blutzucker senkende Wirkung entfalten. Diese Verbindungen sollen sich insbesonders zur Behandlung von Diabetes eignen.

Die Erfindung betrifft daher Verbindungen der Formel I, 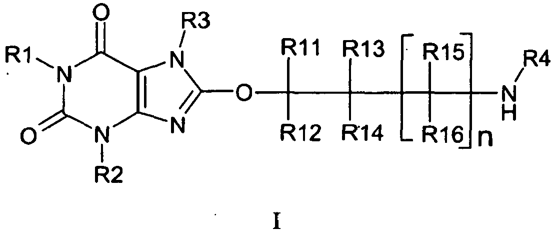 worin bedeuten
- R1, R2, R3: unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, NO₂, SF₅, OH, (C₁-C₆)-Alkyl, -CF₃, -OCF₃; -SCF₃, (C₂-C₆)-Alkenyl, (C₂-C₆)-Alkinyl, OR7, OP(O)(OR7)₂, NR7R8, NR7CONR7R8, COR7, OCOR7, OCOOR7, COOR7, CONR7R8, OCONR7R8, (C₁-C₆)-Alkylen-OR7, (C₁-C₆)-Alkylen-NR7R8, (C₁-C₆)-Alkylen-NR7SO₂R7, (C₁-C₆)-Alkylen-SR7, Alkylen-S(O)R7, Alkylen-CONR7R8, SR7, SOR7, SO₂R7, SO₂NR7R8, NR7SO₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl; wobei nicht alle Reste R1, R2 und R3 gleichzeitig Wasserstoff oder Methyl sein dürfen;
- R7, R8: unabhängig voneinander H, (C₁-C₆)-Alkyl, (C₃-C₁₀)-Cycloalkyl, Aryl, Heterocyclyl, (C₁-C₄)-Alkylen-(C₆-C₁₀)-Aryl oder (C₁-C₄)-Alkylen-Heterocyclyl;
- R4: Wasserstoff, (C₁-C₆)-Alkyl oder (C₃-C₈)-Cykloalkyl, wobei (C₁-C₆)-Alkyl oder (C₃-C₈)-Cykloalkyl substitiuert sein können mit F, Cl, Br, I, CN, Aryl, Heterocyclyl, NH₂, NH(C₁-C₆)-Alkyl, N((C₁-C₆)-Alkyl)₂, OH, O(C₁-C₆)-Alkyl, OAryl, OHeteroaryl, S(C₁-C₆)-Alkyl, S(O)(C₁-C₆)-Alkyl, S(O)₂(C₁-C₆)-Alkyl;
- R11, R12, R13, R14, R15, R16: unabhängig voneinander H, (C₁-C₆)-Alkyl, (C₃-C₈)-Cykloalkyl, (C₁-C₄)-Alkylen-Aryl oder (C₁-C₄)-Alkylen-Heterocyclyl;
oder zwei der Reste R4, R11, R12, R13, R14, R15, R16 bilden gemeinsam einen (C₂-C₆)-Alkylenrest;
- n: 0;
wobei die Verbindung 1,3-Di(cyclopropylmethyl)-7-(4-methoxybenzyl-)-8-(4-piperidinyloxy)xanthine ausgenommen ist,
sowie deren physiologisch verträglichen Salze.

Weiterhin besonders bevorzugt sind Verbindungen der Formel I, worin ein oder mehrere Reste die folgende Bedeutung haben:
- R1, R2, R3: unabhängig voneinander (C₁-C₆)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, CN, NO₂, SF₅, OH, (C₁-C₆)-Alkyl, -CF₃, -OCF₃, - SCF₃, (C₂-C₆)-Alkenyl, (C₂-C₆)-Alkinyl, OR7, NR7R8, NR7CONR7R8, COR7, COOR7, CONR7R8, (C₁-C₆)-Alkylen-OR7, (C₁-C₆)-Alkylen-NR7R8, (C₁-C₆)-Alkylen-NR7SO₂R7, (C₁-C₆)-Alkylen-SR7, Alkylen-S(O)R7, Alkylen-CONR7R8, SR7, SOR7, SO₂R7, SO₂NR7R8, NR7SO₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl; wobei nicht alle Reste R1, R2 und R3 gleichzeitig Methyl sein dürfen;
- R7, R8: unabhängig voneinander H, (C₁-C₆)-Alkyl, (C₃-C₁₀)-Cycloalkyl, Aryl, Heterocyclyl (C₁-C₄)-Alkylen-(C₆-C₁₀)-Aryl oder (C₁-C₄)-Alkylen-Heterocyclyl;
- R4: Wasserstoff;
- R11, R12, R13, R14: unabhängig voneinander H, (C₁-C₆)-Alkyl oder (C₃-C₈)-Cycloalkyl, (C₁-C₄)-Alkylen-Aryl oder (C₁-C₄)-Alkylen-Heterocyclyl;
- n: 0;
sowie deren physiologisch verträglichen Salze.

Weiterhin besonders bevorzugt sind Verbindungen der Formel I, worin ein oder mehrere Reste die folgende Bedeutung haben:
- R1, R2, R3: unabhängig voneinander (C₁-C₆)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, CN, SF₅, (C₁-C₆)-Alkyl, -CF₃, -OCF₃, -SCF₃, (C₂-C4)-Alkenyl, (C₂-C4)-Alkinyl, OR7, NR7R8, NR7CONR7R8, COR7, COOR7, CONR7R8, (C₁-C4)-Alkylen-OR7, (C₁-C4)-Alkylen-NR7R8, (C₁-C4)-Alkylen-NR7SO₂R7, (C₁-C₆)-Alkylen-SR7, Alkylen-S(O)R7, Alkylen-CONR7R8, SR7, SOR7, SO₂R7, SO₂NR7R8, NR7SO₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl; wobei nicht alle Reste R1, R2 und R3 gleichzeitig Methyl sein dürfen;
- R7, R8: unabhängig voneinander H, (C₁-C₆)-Alkyl, (C₃-C₁₀)-Cycloalkyl, Aryl, Heterocyclyl, (C₁-C₄)-Alkylen-(C₆-C₁₀)-Aryl oder (C₁-C₄)-Alkylen-Heterocyclyl;
- R4: Wasserstoff;
R11, R12, R13, R14 unabhängig voneinander H, (C₁-C4)-Alkyl;
- n: 0;
sowie deren physiologisch verträglichen Salze.

Die Erfindung bezieht sich auf Verbindungen der Formel I, in Form ihrer Racemate, racemischen Mischungen und reinen Enantiomere sowie auf ihre Diastereomere und Mischungen davon.
CONR7R8, SR7, SOR7, SO₂R7, SO₂NR7R8, NR7SO₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl; wobei nicht alle Reste R1, R2 und R3 gleichzeitig Wasserstoff oder Methyl sein dürfen;
- R7, R8: unabhängig voneinander H, (C₁-C₆)-Alkyl, (C₃-C₁₀)-Cycloalkyl, (C₆-C₁₀)-Aryl, Heterocyclyl, (C₁-C₆)-Alkylen-CONR9R10, (C₁-C₆)-Alkylen-COOR9, (C₁-C₆)-Alkylen-COR9, (C₁-C₆)-Alkylen-OR9, (C₁-C₆)-Alkylen-NR9R10, (C₁-C₄)-Alkylen-(C₆-C₁₀)-Aryl oder (C₁-C₄)-Alkylen-Heterocyclyl;
- R9, R10: unabhängig voneinander H, (C₁-C₆)-Alkyl, (C₁-C₆)-Alkylen-(C₆-C₁₀)-Aryl, (C₁-C₆)-Alkylen-Heterocyclyl;
- R4: Wasserstoff, (C₁-C₆)-Alkyl oder (C₃-C₈)-Cykloalkyl, wobei (C₁-C₆)-Alkyl oder (C₃-C₈)-Cykloalkyl substitiuert sein können mit F, Cl, Br, I, CN,Aryl, Heterocyclyl, NH₂, NH(C₁-C₆)-Alkyl, N((C₁-C₆)-Alkyl)₂, OH, O(C₁-C₆)-Alkyl, OAryl, OHeteroaryl, S(C₁-C₆)-Alkyl, S(O)(C₁-C₆)-Alkyl, S(O)₂(C₁-C₆)-Alkyl; wobei die Aryl- und Heterocyclylreste ein- oder mehrfach substituiert sein können mit F, Cl, Br, I, CN, NO₂, SF₅, OH, (C₁-C₆)-Alkyl, -CF₃, -OCF₃, -SCF₃, (C₂-C₆)-Alkenyl, (C₂-C₆)-Alkinyl, O(C₁-C₄)-Alkyl, NH₂, NH(C₁-C₄)-Alkyl, N((C₁-C₄)-Alkyl)₂, NR7CONH(C₁-C4)-Alkyl , CO(C₁-C₄)-Alkyl, OCO(C₁-C₄)-Alkyl, OCOO(C₁-C₄)-Alkyl, COO(C₁-C₄)-Alkyl, CONH₂, CONH(C₁-C₄)-Alkyl, CON((C₁-C₄)-Alkyl)₂, (C₁-C₆)-Alkylen-O(C₁-C₄)-Alkyl, (C₁-C₆)-Alkylen-NH(C₁-C₄)-Alkyl, (C₁-C₆)-Alkylen-NH₂, (C₁-C₆)-Alkylen-N((C₁-C₄)-Alkyl)₂, (C₁-C₆)-Alkylen-NHSO₂(C₁-C₄)-Alkyl, (C₁-C₆)-Alkylen-S(C₁-C₄)-Alkyl, Alkylen-S(O)-(C1-C4)-Alkyl, Alkylen-CONH₂, Alkylen-CONH(C₁-C₄)-Alkyl, Alkylen-CON((C₁-C₄)-Alkyl)₂, S(C₁-C₄)-Alkyl, SO(C₁-C₄)-Alkyl, SO₂(C₁-C₄)-Alkyl, SO₂NH₂, SO₂NH(C₁-C₄)-Alkyl, SO₂N((C₁-C₄)-Alkyl)₂, NR7SO₂(C₁-C₄)-Alkyl , (C₁-C₆)-Alkylen-(C₃-C₁₀)-Cycloalkyl, (C₃-C₁₀)-Cycloalkyl;

- R11, R12, R13, R14, R15, R16: unabhängig voneinander H, (C₁-C₆)-Alkyl, (C₃-C₈)-Cykloalkyl, (C₁-C₄)-Alkylen-OAlkyl, (C₁-C₄)-Alkylen-SAlkyl, (C₁-C₄)-Alkylen-NHAlkyl, (C₁-C₄)-Alkylen-N(Alkyl)₂, (C₁-C₄)-Alkylen-Aryl, (C₁-C₄)-Alkylen-Heterocyclyl, F, Cl, Br, I, CN, COOH, COO(C₁-C₆)-Alkyl, CONH₂, CONH(C₁-C₆)-Alkyl, CON((C₁-C₆)-Alkyl)₂, CF₃;
oder zwei der Reste R4, R11, R12, R13, R14, R15, R16 bilden gemeinsam einen (C₂-C₆)-Alkylenrest; wobei der (C₂-C₆)-Alkylenrest ein- oder mehrfach substituiert sein kann mit F, Cl, Br, I, CN, NO₂, SF₅, OH, (C₁-C₆)-Alkyl, -CF₃, -OCF₃, -SCF₃, . (C₂-C₆)-Alkenyl, (C₂-C₆)-Alkinyl, O(C₁-C₄)-Alkyl, NH₂, NH(C₁-C₄)-Alkyl, N((C₁-C₄)-Alkyl)₂, NR7CONH(C₁-C₄)-Alkyl, CO(C₁-C₄)-Alkyl, OCO(C₁-C₄)-Alkyl, OCOO(C₁-C₄)-Alkyl, COO(C₁-C₄)-Alkyl, CONH₂, CONH(C₁-C₄)-Alkyl, CON((C₁-C₄)-Alkyl)₂, (C₁-C₆)-Alkylen-O(C₁-C₄)-Alkyl, (C₁-C₆)-Alkylen-NH(C₁-C₄)-Alkyl, (C₁-C₆)-Alkylen- NH₂, (C₁-C₆)-Alkylen-N((C₁-C₄)-Alkyl)₂, (C₁-C₆)-Alkylen-NHSO₂(C,-C₄)-Alkyl, (C₁-C₆)-Alkylen-S(C₁-C₄)-Alkyl, Alkylen-S(O)-(C1-C4)-Alkyl, Alkylen-CONH₂, Alkylen-CONH(C₁-C₄)-Alkyl, Alkylen-CON((C₁-C₄)-Alkyl)₂, S(C₁-C₄)-Alkyl, SO(C₁-C₄)-Alkyl, SO₂(C₁-C₄)-Alkyl, SO₂NH₂, SO₂NH(C₁-C₄)-Alkyl, SO₂N((C₁-C₄)-Alkyl)₂, NR7SO₂(C₁-C₄)-Alkyl, (C₁-C₆)-Alkylen-(C₃-C₁₀)-Cycloalkyl, (C₃-C₁₀)-Cycloalkyl;
- n: 0 oder 1;
wobei die Verbindung 1,3-Di(cyclopropylmethyl)-7-(4-methoxybenzyl-)-8-(4-piperidinyloxy)xanthine ausgenommen ist,
sowie deren physiologisch verträglichen Salze.

Besonders bevorzugt sind Verbindungen der Formel I, worin ein oder mehrere Reste die folgende Bedeutung haben:
- R1, R2, R3: unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, NO₂, SF₅, OH, (C₁-C₆)-Alkyl, -CF₃, -OCF₃, -SCF₃, (C₂-C₆)-Alkenyl, (C₂-C₆)-Alkinyl, OR7, OP(O)(OR7)₂, NR7R8, NR7CONR7R8, COR7, OCOR7, OCOOR7, COOR7, CONR7R8, OCONR7R8, (C₁-C₆)-Alkylen-OR7, (C₁-C₆)-Alkylen-NR7R8, (C₁-C₆)-Alkylen-NR7SO₂R7, (C₁-C₆)-Alkylen-SR7, Alkylen-S(O)R7, Alkylen-CONR7R8, SR7, SOR7, SO₂R7, SO₂NR7R8, NR7SO₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl; wobei nicht alle Reste R1, R2 und R3 gleichzeitig Wasserstoff oder Methyl sein dürfen;
- R7, R8: unabhängig voneinander H, (C₁-C₆)-Alkyl, (C₃-C₁₀)-Cycloalkyl, Aryl, Heterocyclyl, (C₁-C₄)-Alkylen-(C₆-C₁₀)-Aryl oder (C₁-C₄)-Alkylen-Heterocyclyl;
- R4: Wasserstoff, (C₁-C₆)-Alkyl oder (C₃-C₈)-Cykloalkyl, wobei (C₁-C₆)-Alkyl oder (C₃-C₈)-Cykloalkyl substitiuert sein können mit F, Cl, Br, I, CN, Aryl, Heterocyclyl, NH₂, NH(C₁-C₆)-Alkyl, N((C₁-C₆)-Alkyl)₂, OH, O(C₁-C₆)-Alkyl, OAryl, OHeteroaryl, S(C₁-C₆)-Alkyl, S(O)(C₁-C₆)-Alkyl, S(O)₂(C₁-C₆)-Alkyl;
- R11, R12, R13, R14, R15, R16: unabhängig voneinander H, (C₁-C₆)-Alkyl, (C₃-C₈)-Cykloalkyl, (C₁-C₄)-Alkylen-Aryl oder (C₁-C₄)-Alkylen-Heterocyclyl;
oder zwei der Reste R4, R11, R12, R13, R14, R15, R16 bilden gemeinsam einen (C₂-C₆)-Alkylenrest;
- n: 0;
wobei die Verbindung 1,3-Di(cyclopropylmethyl)-7-(4-methoxybenzyl-)-8-(4-piperidinyloxy)xanthine ausgenommen ist,
sowie deren physiologisch verträglichen Salze.

Weiterhin besonders bevorzugt sind Verbindungen der Formel I, worin ein oder mehrere Reste die folgende Bedeutung haben:
- R1, R2, R3: unabhängig voneinander (C₁-C₆)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, CN, NO₂, SF₅, OH, (C₁-C₆)-Alkyl, -CF₃, -OCF₃, - SCF₃, (C₂-C₆)-Alkenyl, (C₂-C₆)-Alkinyl, OR7, NR7R8, NR7CONR7R8, COR7, COOR7, CONR7R8, (C₁-C₆)-Alkylen-OR7, (C₁-C₆)-Alkylen-NR7R8, (C₁-C₆)-Alkylen-NR7SO₂R7, (C₁-C₆)-Alkylen-SR7, Alkylen-S(O)R7, Alkylen-CONR7R8, SR7, SOR7, SO₂R7, SO₂NR7R8 NR7SO₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl; wobei nicht alle Reste R1, R2 und R3 gleichzeitig Methyl sein dürfen;
- R7, R8: unabhängig voneinander H, (C₁-C₆)-Alkyl, (C₃-C₁₀)-Cycloalkyl, Aryl, Heterocyclyl (C₁-C₄)-Alkylen-(C₆-C₁₀)-Aryl oder (C₁-C₄)-Alkylen-Heterocyclyl;
- R4: Wasserstoff;
- R11, R12, R13, R14: unabhängig voneinander H, (C₁-C₆)-Alkyl oder C3-C7-Cycloalkyl, (C₁-C₄)-Alkylen-Aryl oder (C₁-C₄)-Alkylen-Heterocyclyl;
- n: 0;
sowie deren physiologisch verträglichen Salze.

Weiterhin besonders bevorzugt sind Verbindungen der Formel I, worin ein oder mehrere Reste die folgende Bedeutung haben:
- R1, R2, R3: unabhängig voneinander (C₁-C₆)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, CN, SF₅, (C₁-C₆)-Alkyl, -CF₃, -OCF₃, -SCF₃, (C₂-C4)-Alkenyl, (C₂-C4)-Alkinyl, OR7, NR7R8, NR7CONR7R8, COR7, COOR7, CONR7R8, (C₁-C4)-Alkylen-OR7, (C₁-C4)-Alkylen-NR7R8, (C₁-C4)-Alkylen-NR7SO₂R7, (C₁-C₆)-Alkylen-SR7, Alkylen-S(O)R7, Alkylen-CONR7R8, SR7, SOR7, SO₂R7, SO₂NR7R8, NR7SO₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl; wobei nicht alle Reste R1, R2 und R3 gleichzeitig Methyl sein dürfen;
- R7, R8: unabhängig voneinander H, (C₁-C₆)-Alkyl, (C₃-C₁₀)-Cycloalkyl, Aryl, Heterocyclyl, (C₁-C₄)-Alkylen-(C₆-C₁₀)-Aryl oder (C₁-C₄)-Alkylen-Heterocyclyl;
- R4: Wasserstoff;
- R11, R12, R13, R14: unabhängig voneinander H, (C₁-C4)-Alkyl;
- n: 0;
sowie deren physiologisch verträglichen Salze.

Die Erfindung bezieht sich auf Verbindungen der Formel I, in Form ihrer Racemate, racemischen Mischungen und reinen Enantiomere sowie auf ihre Diastereomere und Mischungen davon.

Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroximethyl-1,3-propandiol), Diethanolamin, Lysin, oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Alkylrest wird eine geradkettige oder verzweigte Kohlenwasserstoffkette mit einem oder mehreren Kohlenstoffen verstanden, wie z.B. Methyl, Ethyl, iso-Propyl, tert.-Butyl, Hexyl.
Die Alkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, , SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heteroeyclus)-CO-NH-Aryl, N(Aryl)-CO-N-(C₁-C₆)-Alkyl)₂, N(Heteroeyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl.
Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂,, SO₂-N((CH₂)ₙ-(Heterocyclus)₂ wobei n =0-6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N( (C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂) NH(C₁-C₆)-Alkyl, N((C₁-C₆)-Alkyl)₂, SO₂-CH₃, COOH, COO-(C₁-C₆)-Alkyl, CONH₂.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Hexinyl.
Die Alkinylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₁-C₁₀)-Alkyl, O-(C₁-C₆)-Alkyl O-CO-(C₁-C₆)-Alkyl, O-CO-(C₁-C₆)-Aryl, O-CO-(C₁-C₆)-Heterocyclus;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂),-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, S0₂-N((CH₂)ₙ-Aryl)₂, , SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.
Die Arylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂,, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.
Die Cycloalkylrestereste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus,; PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂,, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter Heterocyclyl bzw. Heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der Heterocyclische Rest mit Benzolkernen kondensiert ist.

Geeignete Heterocyclische Ringe, Heterocyclyl bzw. Heterocyclische Reste sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxi-2-, 3- oder 4-pyridyl.

Umfasst sind weiterhin ein oder mehrfach benzoannelierte Derivate dieser Heterocyclen.

Die Heterocyclischen Ringe, Heterocyclyl bzw. Heterocyclische Reste können ein oder mehrfach mit geeigneten Gruppen substituiert sein, wie z.B: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₁-C₁₀)-Alkyl, (C₂-C₆)-Alkenyl, (C₂-C₆)-Alkinyl, O-(C₁-C₆)-Alkyl O-CO-(C₁-C₆)-Alkyl, O-CO-(C₁-C₆)-Aryl, O-CO-(C₁-C₆)-Heterocyclus;
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂,, SO₂-N((CH₂)ₙ-(Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
C(NH)(NH₂), NH₂, NH-(C₁-C₆)-Alkyl, N((C₁-C₆)-Alkyl)₂, NH(C₁-C₇)-Acyl, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N(C₁-C₆)-Alkyl -CO-(C₁-C₆)-Alkyl, N(C₁-C₆)-Alkyl -COO-(C₁-C₆)-Alkyl, N(C₁-C₆)-Alkyl - CO-Aryl, N(C₁-C₆)-Alkyl -CO-Heterocyclus, N(C₁-C₆)-Alkyl -COO-Aryl, N(C₁-C₆)-Alkyl - COO-Heterocyclus, N(C₁-C₆)-Alkyl -CO-NH-(C₁-C₆)-Alkyl), N(C₁-C₆)-Alkyl -CO-NH-Aryl, N(C₁-C₆)-Alkyl -CO-NH-Heterocyclus, N((C₁-C₆)-Alkyl)-CO-N-(C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N-(C₁-C₆)-Alkyl)₂, N(Heterocyclus)-CO-N-(C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Die Verbindung(en) der Formel (I) können auch in Kombination mit weiteren Wirkstoff verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxipropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontoforese freigesetzt werden.

Als weitere Wirkstoffe für die Kombinationspräparate sind geeignet:
Alle Antidiabetika, die in der Roten Liste 2004, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Alle Antidiabetika, die in der Roten Liste 2004, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.
Die oral wirksamen hypoglykämischen Wirkstoffe umfassen vorzugsweise Sulfonylfhamstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, oder mit einer Verbindung, wie in PCT/EP 2003/05815, PCT/EP 2003/05814, PCT/EP 2003/05816, US 6,498,156 beschrieben, verabreicht.

Bei einer Ausführungsform der Erfindung werden die Verbindungen der Formel I in Kombination mit einem gemischten PPAR alfa/gamma Agonisten, wie z.B. GW 1536, AVE 8042, AVE 8134, AVE 0847, oder wie in PCT/US 2000/11833, PCT/US 2000/11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsfbrm der Erfindung werden die Verbindungen der Formel I in Kombination mit einem ATP-Citrat-Lyase Inhibitor, wie z.B. SB-204990, verabreicht.

Bei einer Ausfiihrungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulfonylharnstoff, wie z.B. Tolbutamid, Glibenclamid, Glipizid oder Glimepirid verabreicht. Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxi]phenyl]methyl]-2,4-thiazolidindion, verabreicht.
Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit einem Adenosin A1 Agonisten, wie z. B. solchen, die in EP 0912520 beschrieben sind, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure- {4-[(4-amino-quinazolin-2-ylamino)-methyl]-cyclohexylmethyl} -amid Hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-(Melanocyt-stimulierendes Hormon)-Agonisten, CCK-A Agonisten (z.B. {2-[4-(4-Chloro-2,5-dimethoxi-phenyl)-5-(2-cyclohexyl-ethyl)-thiazol-2-ylcarbamoyl]-5,7-dimethyl-indol-1-yl}-essigsäure Trifluoressigsäuresalz (WO 99/15525)), Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxi-1-(2-diisopropylamino-ethylcarbamoyl)-3,4-dihydro-1H-isochinolin-2-carbonsäuretertiärbutylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.
Bei einer Ausführungsform ist der weitere Wirkstoff Fenfluramin oder Dexfenfluramin.
Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer weiteren Ausführungsform ist der weitere Wirkstoff Rimonabant.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

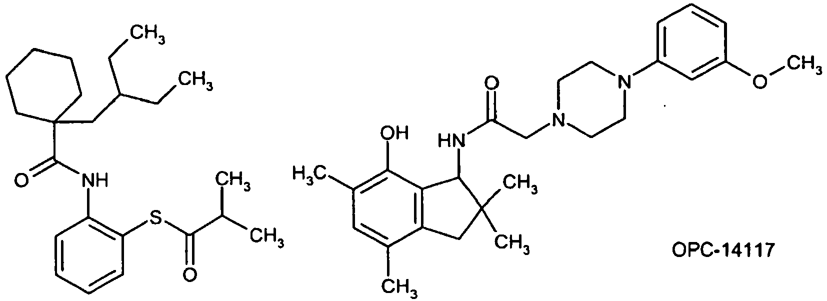

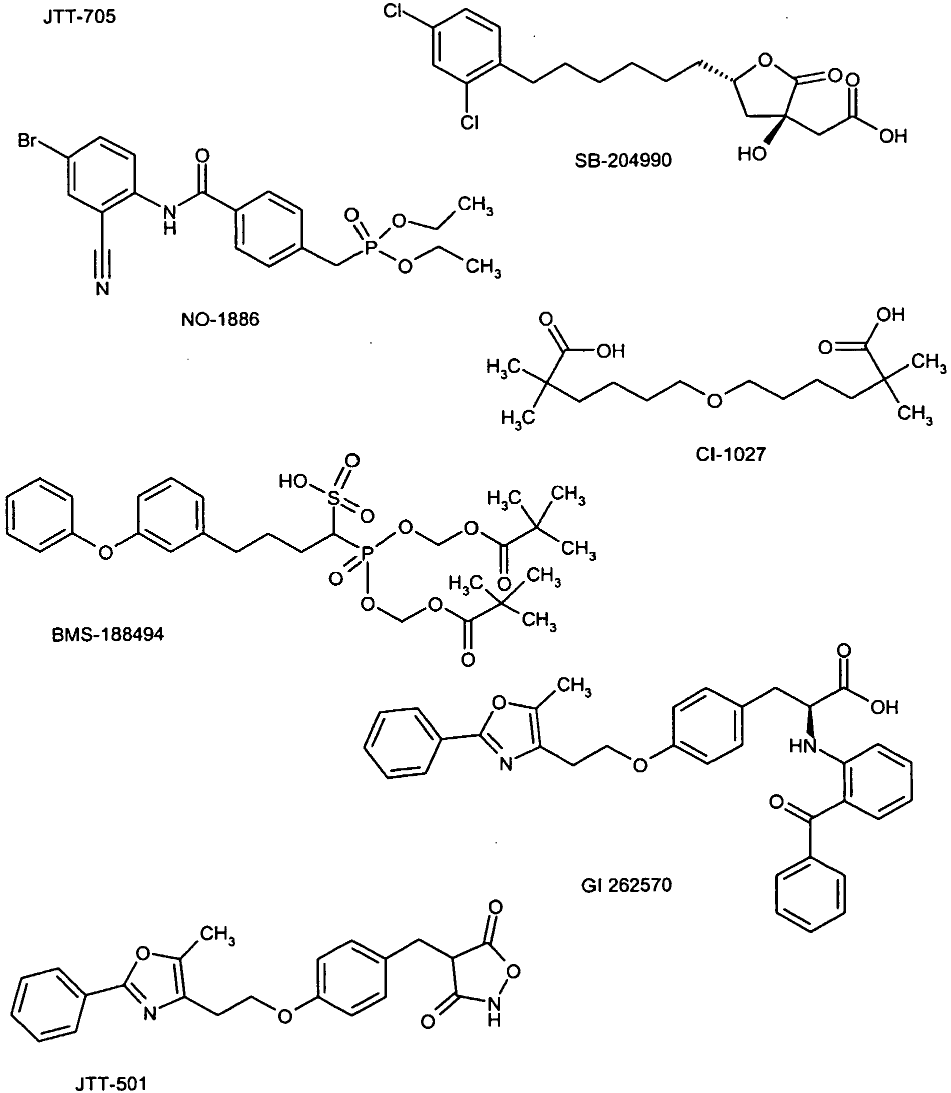

Die Verbindungen der Formel I lassen sich dadurch herstellen, dass man geeignete Ausgangsstoffe der Formel II, worin R1, R2 und R3 die oben angegebene Bedeutung besitzen und X eine Austrittsgruppe, wie Chlor, Brom, Jod, Sulfonyloxi, Sulfinyl, Sulfoxil, bedeutet, mit einer Verbindung der Formel III, wobei M für ein Metall wie Lithium, Natrium, Kalium, Cäsium, Magnesium, Calcium usw. steht, ggf. in Gegenwart von geeigneten Basen in inerten Lösemitteln zu den Verbindungen der Formel I umsetzt.
Alternativ kann man die Ausgangsstoffe der Formel II mit Alkoholen der Formel III, worin M die Bedeutung von Wasserstoff besitzt, in Gegenwart von geeigneten Basen in inerten Lösemitteln zu den erfindungsgemäßen Stoffen der Formel I umsetzen.

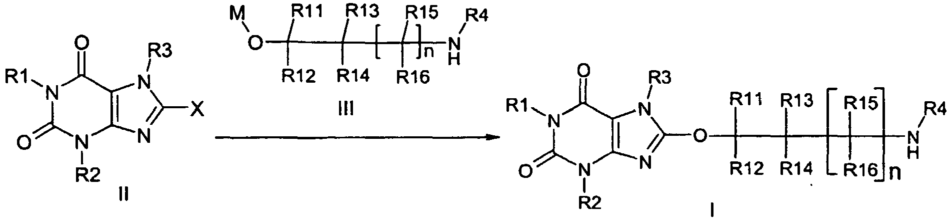

In den Fällen, wo R4 Wasserstoff bedeutet, kann es zweckmäßig sein, den Rest -NHR4 in an der Stickstofffunktion geschützter Form einzusetzen und die Schutzgruppe an geeigneter Stelle der Reaktion wieder abzuspalten. Solche geeignete Schutzgruppen und die Verfahren der Einführung und Abspaltung sind bekannt (Siehe:Theodora W. Greene and Peter G. M. Wuts, Protective Groups in Organic Synthesis, 3rd edition, John Wiley & Sons, Inc., New York, 1999 )

Die Halogenverbindungen der Formel II können nach bekannten Verfahren wie beispielsweise durch Halogenierung der entsprechenden H-oder OH-Verbindung (Formel 2, X = H, OH) erhalten werden. Geeignete Halogenierungsmittel können beispielhaft Halogene, wie Chlor und Brom, N-Bromsuccinimid, Phosphorpentoxid oder Phosphoroxichlorid sein.

Die Synthese von Verbindungen der Formel II ist mehrfach in der Literatur beschrieben (siehe Houben Weyl E9b/2, S. 331 ff). Sie können beispielsweise ausgehend von Diaminopyrimidinderivaten oder Aminoimidazolcarbonsäureamiden durch Umsetzung mit geeigneten Reagentien erhalten und durch gezielte chemische Modifikationen wie Hydrolyse, Alkylierung, Halogenierung in die gewünschten Ausgangsverbindungen der Formel II umgewandelt werden.

Die Reste R1 bis R3 lassen sich nach an sich bekannten Methoden durch Alkylierung entsprechender Vorstufen herstellen, wobei die Reihenfolge variiert werden kann. Zum Teil können sie aber auch durch die Auswahl entsprechender Vorstufen bei der Herstellung des Xanthingerüstes eingeführt werden.

Die Verbindungen der Formel III sind bekannt oder lassen sich nach literaturbekannten Verfahren herstellen.

**Tabelle1 :**

| | | | | | | | | | | | |
|---|---|---|---|---|---|---|---|---|---|---|---|
| 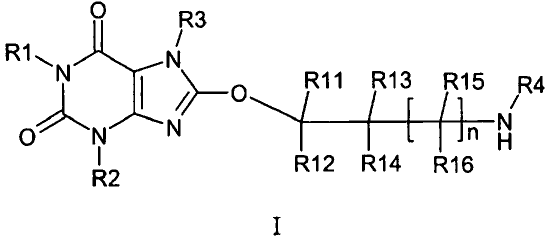 | | | | | | | | | | | |

| Bsp. | R1 | R2 | R3 | R4 | R11 | R12 | R13 | R14 | n | R15 | R16 |
|---|---|---|---|---|---|---|---|---|---|---|---|
| 1 | Ph-CO-CH₂- | CH₃ | -CH₂CH=C(CH₃)₂ | H | H | H | CH₃ | CH₃ | 0 | - | - |
| 2 | Ph-CO-CH₂- | CH₃ | -CH₂CH=C(CH₃)₂ | H | H | H | H | H | 0 | - | - |
| 3 | Ph-CO-CH₂- | CH₃ | -CH₂CH=C(CH₃)₂ | H | H | H | CH₂-Ph-4-Cl | H | 0 | - | - |
| 4 | Ph-CO-CH₂- | CH₃ | -CH₂CH=C(CH₃)₂ | H | H | H | CH₃ | H | | - | - |
| 5 | Ph-CO-CH₂- | CH₃ | -CH₂CH=C(CH₃)₂ | | R₄+R₁₁= (CH₂)₃ | H | H | H | 0 | - | - |
| 6 | Ph-CO-CH₂- | CH₃ | -CH₂CH=C(CH₃)₂ | H | H | H | CH₃ | CH₃ | 1 | H | H |
| 7 | Ph-CO-CH₂- | CH₃ | -CH₂CH=C(CH₃)₂ | | H | H | R₄+R)₃= (CH₂)₃ | H | 0 | - | - |
| 8 | Ph-CO-CH₂- | CH₃ | -CH₂CH=C(CH₃)₂ | | H | H | (S)-R₄+R₁₃=(CH₂)₂ | H | 0 | - | - |
| 9 | Ph-CO-CH₂- | CH₃ | -CH₂CH=C(CH₃)₂ | | H | H | (S)-R₄⁺R₁₃= (CH₂)₂ | H | 1 | H | H |
| 10 | Ph-CO-CH₂- | CH₃ | -CH₂CH=C(CH₃)₂ | | H | H | (R)-R₄+R₁₃= (CH₂)₂ | H | 1 | H | H |
| 11 | Ph-CO-CH₂- | CH₃ | -CH₂CH=C(CH₃)₂ | H | H | H | (R)-CH₃ | H | 0 | - | - |
| 12 | Ph-CO-CH₂- | CH₃ | -CH₂CH=C(CH₃)₂ | H | H | H | H | H | 1 | H | H |
| 13 | Ph-CO-CH₂- | CH₃ | -CH₂CH=C(CH₃)₂ | H | CH₃.HCl | H | H | H | 0 | - | - |
| 14 | Ph-CO-CH₂- | CH₃ | -CH₂CH=C(CH₃)₂ | H | R₁₁⁺R₁₃ = (CH₂)₄ (trans) | H | | H | 0 | - | - |
| 15 | Ph-CO-CH₂- | CH₃ | -CH₂CH=C(CH₃)₂ | H | R₁₁+R₁₃ = (CH₂)₄(cis) | H | | H | 0 | - | - |
| 16 | Ph-CO-CH₂- | CH₃ | -CH₂-(2-Cl,4-F-Ph) | H | H | H | H | H | 0 | - | - |
| 17 | Ph-CO-CH₂- | CH₂-CH₃ | -CH₂CH=C(CH₃)₂ | H | H | H | H | H | 0 | - | - |
| 18 | Ph-CO-CH₂- | CH₃ | -CH2-CC-CH₃ | H | H | H | H | H | 0 | - | - |
| 19 | Ph-CO-CH₂- | CH₃ | -CH₂CH=C(CH₃)₂ | H | R₁₁⁺R₁₃ = indan-1,2-diyl (1S,2R) | H | | H | 0 | - | - |
| 20 | Ph-CO-CH₂- | CH₃ | -CH₂CH=C(CH₃)₂ | H | H | H | CH₂-Ph Trifluoracetat | H | 0 | - | - |
| 21 | Ph-CO-CH₂- | CH₃ | -CH₂CH=C(CH₃)₂ | H | R₁₁+R₁₃ = indan-1,2-diyl (1R,2S) | H | Trifluoracetat | H | 0 | - | - |
| 22 | CH₃ | CH₃ | -CH₂-(2-CN-Ph) | H | H | H | H | H | 0 | - | - |
| 23 | CH₃ | CH₃ | -CH₂-CH=CH-Ph | H | H | H | H | H | 0 | - | - |
| 24 | Ph-CO-CH₂- | CH₂-CF₃ | -CH₂CH=C(CH₃)₂ | H | H | H | H.HCl | H | 0 | - | - |
| 25 | Ph-CO-CH₂- | CH₃ | -CH₂CH=C(CH₃)₂ | | H | H | (R)-R₄+R₁₃= (CH₂)₂ | H | 0 | - | - |
| 26 | Ph-CO-CH₂- | CH₃ | -CH₂CH=C(CH₃)₂ | | (R)-R₄+R₁₁= (CH₂)₂ | H | H | H | 0 | - | - |
| 27 | Ph-CO-CH₂- | CH₃ | -CH₂CH=C(CH₃)₂ | | (S)-R₄+R₁₁= (CH₂)₂ | H | H | H | 0 | - | - |
| 28 | Ph-CO-CH₂- | CH₃ | -CH₂CH=C(CH₃)₂ | H | R₁₁+R₁₃= (CH₂)₄ cis | H | | H | 1 | H | H |
| 29 | Ph-CO-CH₂- | CH₃ | -CH₂CH=C(CH₃)₂ | H | H | H | R₁₃+R₁₅= (CH₂)₄ trans | H | 1 | | H |
| 30 | Ph-CO-CH₂- | CH₃ | -CH₂CH=C(CH₃)₂ | H | H | H | R₁₃+R₁₅= (CH₂)₄ cis | H | 1 | | H |
| 31 | Ph-CO-CH₂- | CH₃ | -CH₂CH=C(CH₃)₂ | H | R₁₁+R₁₃= (CH₂)₃ trans | H | | H | 0 | - | - |
| 32 | Ph-CO-CH₂- | CH₃ | -CH₂CH=C(CH₃)₂ | H | R₁₁+R₁₃= (CH₂)₄ trans | H | Hydrochlorid | H | 0 | - | - |
| 33 | Ph-CO-CH₂- | CH₃ | -CH₂CH=C(CH₃)₂ | H | H | H | CH₃.HCl | CH₃ | 1 | H | H |
| 34 | Ph-CO-CH₂- | CH₃ | -CH₂CH=C(CH₃)₂ | | H | H | CH₃ | H | 1 | H | H |
| 35 | Ph-CO-CH₂- | CH₃ | -CH₂CH=C(CH₃)₂ | | H R₁₁+R₁₃= (CH₂)₄ trans | H | | H | 1 | H | H |
| 36 | Ph-CO-CH₂- | CH₃ | -CH₂CH=C(CH₃)₂ | H | R₁₁+R₁₃= (CH₂)₄ trans | H | Hydrochlorid | H | 1 | H | H |
| 37 | Ph-CO-CH₂- | CH₃ | -CH₂CH=C(CH₃)₂ | | H R₁₁+R₁₃= (CH₂)₄ trans | H | Diastereomer 1 | H | 1 | H | H |
| 38 | Ph-CO-CH₂- | CH₃ | -CH₂CH=C(CH₃)₂ | | H R₁₁+R₁₃= (CH₂)₄ trans | H | Diastereomer 2 | H | 1 | H | H |
| 39 | Ph-CO-CH₂- | CH₃ | -CH₂CH=C(CH₃)₂ | H | R₁₁+R₁₃= (CH₂)₄ trans | H | Hydrochlorid Diastereomer 1 | H | 0 | - | - |
| 40 | Ph-CO-CH₂- | CH₃ | -CH₂CH=C(CH₃)₂ | H | R₁₁⁺R₁₃= (CH₂)₄ trans | H | Hydrochlorid Diastereomer 2 | H | 0 | - | - |
| 41 | CH₃ | CH₃ | -CH₂-(2-Cl-Ph) | H | R₁₁+R₁₃= (CH₂)₄ trans | H | | H | 0 | - | - |

Die Verbindungen der Formel I zeichnen sich durch günstige Wirkungen auf den Lipid-und Kohlenhydratstoffwechsel aus, sie senken insbesondere den Blutzuckerspiegel und sind zur Behandlung von Typ 2 Diabetes, von Insulinresistenz, von Dyslipidämien und des metabolischen Syndroms / Syndrom X geeignet. Weiterhin sind die Verbindungen zur Prophylaxe und Behandlung von arteriosklerotischen Erscheinungen geeignet. Die Verbindungen können allein oder in Kombination mit weiteren Blutzucker senkenden Wirkstoffen eingesetzt werden. Die Verbindungen wirken als DPP-IV (Dipeptidyl Peptidase IV) Inhibitoren und eignen sich auch zur Behandlung von Störungen des Empfindens und anderer psychiatrischen Indikationen, wie zum Beispiel Depressionen, Angstzuständen, Angstneurosen, Schizophrenie sowie zur Behandlung von Störungen assoziiert mit dem zirkadianen Rhythmus, zur Gewichtsreduktion bei Säugetieren, zur Behandlung von Immunstörungen, und zur Behandlung von Drogenmissbrauch.
Weiterhin eignen sie sich zur Behandlung von Krebs, Arthritis, Osteoarthritis, Osteoporose, Schlafstörungen, Schlaf Apnoe, weiblicher und männlicher Sexualstörungen, Entzündungen, Akne, Pigmentierung der Haut, Störungen des Steroidstoffwechsels, Hautkrankheiten, Psoriasis, Mykosen, neurodegenerativer Krankheiten, Multiple Sklerose und Alzheimer-Krankheit.

Die Wirksamkeit der Verbindungen wurde wie folgt getestet:
Messung der DPP-IV Aktivität:

### Material :

DPP-IV aus Schweineniere (Sigma, München)
H-Ala-Pro-AFC (Bachem, Weil am Rhein)

### Testbedingungen:

DPP-IV (1 mU/ml, Endkonzentratio)
H-Ala-Pro-AFC (15µM Endkonzentration)
in Tris/HCl (40 mM, pH 7.4), Gesamtvolumen 0,2 ml

Die Reaktion wurde bei Raumtemperatur für unterschiedliche Zeiträume (typischerweise 10 min) durchgeführt und am Ende der Reaktion durch Zugabe von 20µl ZnCl₂ (1 M) gestoppt. Der Umsatz von H-Ala-Pro-AFCwurde fluorimetrisch durch Messung der Emission bei 535nm nach Anregung bei 405 nm bestimmt. Im Falle der Zugabe von Inhibitoren wurde das zugegebene Puffervolumen so angepasst, dass ein Gesamtvolumen der Testmischung von 200µl eingehalten wurde.

IC50 Werte für Inhibitoren wurden durch Variation der Inhibitorkonzentrationen bei der angegebenen Substratkonzentration von 15µM bestimmt. Ki und Km Werte wurden durch enstsprechende Variation von Substrat- und Inhibitorkonzentration wie beschrieben (Dixon, M. and Webb, E.C.(1979) Enzymes , third edition, pp. 47-206, Academic Press) ermittelt. Die Werte für Km, IC50 and Ki wurden mit einem kommerziell erhältlichen Software-Paket errechnet (Leatherbarrow, R.J. (1992) GraFit Version 3.0, Erithacus Software Ltd. Staines, U.K.).

**Tabelle 2: Biologische Aktivität**

| Ausführungsbeispiel Nr. | IC-50 (nM) |
|---|---|
| 1 | 92 |
| 2 | 20 |
| 3 | 460 |
| 4 | 28 |
| 7 | 51 |
| 9 | 120 |
| 10 | 34 |
| 11 | 100 |
| 26 | 84 |
| 28 | 13 |
| 37 | 4,5 |

Aus der Tabelle ist abzulesen, dass die Verbindungen der Formel I die Aktivität der DPP-IV (Dipeptidyl Peptidase IV) hemmen und dadurch zur Senkung des Blutzuckerspiegels gut geeignet sind.

Nachfolgend wird die Herstellung einiger Beispiele detailliert beschrieben, die übrigen Verbindungen der Formel I wurden analog erhalten:

### Beispiel 1

8-(2-Amino-2-methyl-propoxi)-3-methyl-7-(3-methyl-but-2-enyl)-1-(2-oxo-2-phenyl-ethyl)-3,7-dihydro-purin-2,6-dion

Zur Lösung von 25 mg 2-Amino-2-methyl-propan-1-ol in 2 ml THF (abs) wurden 15 mg NaH (50%ig in Paraffinöl) gegeben und die Mischung 30 Minuten unter Argon gerührt. Dann wurden 100 mg 8-Brom-3-methyl-7-(3-methyl-but-2-enyl)-1-(2-oxo-2-phenyl-ethyl)-3,7-dihydro-purin-2,6-dion zugefügt und die Mischung 15 Stunden bei Raumtemperatur gerührt. Nach Zugabe von 20 ml Wasser wurde das Produkt mit Essigester (2 x je 10 ml) ausgeschüttelt und das Lösemittel am Vakuum abgezogen.

Der ölige Rückstand wurde säulenchromatografisch (Kieselgel, Laufmittel: Methylenchlorid: Methanol = 9:1) gereinigt.
Ausbeute: 45 mg Fp.: Öl MS: M+1 = 440

Analog wurden durch Verwendung entsprechender Aminoalkohole hergestellt:

### Beispiel 2

8-(2-Amino-ethoxi)-3-methyl-7-(3-methyl-but-2-enyl)-1-(2-oxo-2-phenyl-ethyl)-3,7-dihydropurin-2,6-dion MS: M+1 = 412

### Beispiel 3

8-[2-Amino-3-(4-chloro-phenyl)-propoxi]-3-methyl-7-(3-methyl-but-2-enyl)-1-(2-oxo-2-phenyl-ethyl)-3,7-dihydro-purin-2,6-dion MS: M+1 = 537

### Beispiel 4

(S)-8-(2-Amino-propoxi)-3-methyl-7-(3-methyl-but-2-enyl)-1-(2-oxo-2-phenyl-ethyl)-3,7-dihydro-purin-2,6-dion MS: M+1 = 426

### Beispiel 5

3-Methyl-7-(3-methyl-but-2-enyl)-1-(2-oxo-2-phenyl-ethyl)-8-(piperidin-3-yloxy)-3,7-dihydro-purin-2,6-dion-hydrochlorid MS: M+1 = 452

### Beispiel 6

8-(3-Amino-2,2-dimethyl-propoxy)-3-methyl-7-(3-methyl-but-2-enyl)-1-(2-oxo-2-phenylethyl)-3,7-dihydro-purin-2,6-dion MS: M+1 = 454

### Beispiel 7

3-Methyl-7-(3-methyl-but-2-enyl)-1-(2-oxo-2-phenyl-ethyl)-8-(piperidin-2-ylmethoxy)-3,7-dihydro-purin-2,6-dion MS: M+1 = 466

### Beispiel 8

(S)-3-Methyl-7-(3-methyl-but-2-enyl)-1-(2-oxo-2-phenyl-ethyl)-8-(pyrrolidin-2-ylmethoxy)-3,7- dihydro-purin-2,6-dion-hydrochlorid MS: M+1 = 452

### Beispiel 9

) (S)- 3-Methyl-7-(3-methyl-but-2-enyl)-1-(2-oxo-2-phenyl-ethyl)-8-(pyrrolidin-3-ylmethoxy)-3,7- dihydro-purin-2,6-dion-hydrochlorid MS: M+1 = 452

### Beispiel 10

(R)- 3-Methyl-7-(3-methyl-but-2-enyl)-1-(2-oxo-2-phenyl-ethyl)-8-(pyrrolidin-3-ylmethoxy)-3,7- dihydro-purin-2,6-dion-hydrochlorid MS: M+1 = 452

### Beispiel 11

(R)-8-(2-Amino-propoxy)-3-methyl-7-(3-methyl-but-2-enyl)-1-(2-oxo-2-phenyl-ethyl)-3,7-dihydro- purin-2,6-dion MS: M+1 = 426

### Beispiel 12

8-(3-Amino-propoxy)-3-methyl-7-(3-methyl-but-2-enyl)-1-(2-oxo-2-phenyl-ethyl)-3,7-dihydropurin-2,6-dion MS: M+1 = 426

### Beispiel 13

raz-8-(2-Amino-1-methyl-ethoxy)-3-methyl-7-(3-methyl-but-2-enyl)-1-(2-oxo-2-phenyl-ethyl)-3,7- dihydro-purin-2,6-dion-hydrochlorid MS: M+1 = 426

### Beispiel 14

8-(trans-2-Amino-cyclohexyloxy)-3-methyl-7-(3-methyl-but-2-enyl)-1-(2-oxo-2-phenyl-ethyl)-3,7- dihydro-purin-2,6-dion MS: M+1 = 466

### Beispiel 15

8-(cis-2-Amino-cyclohexyloxy)-3-methyl-7-(3-methyl-but-2-enyl)-1-(2-oxo-2-phenyl-ethyl)-3,7- dihydro-purin-2,6-dion MS: M+1 = 466

### Beispiel 16

8-(2-Amino-ethoxy)-7-(2-chlor-4-fluor-benzyl)-1,3-dimethyl-3,7-dihydro-purin-2,6- dion MS: M+1 = 382

### Beispiel 17

8-(2-Amino-ethoxy)-3-ethyl-7-(3-methyl-but-2-enyl)-1-(2-oxo-2-phenyl-ethyl)-3,7-dihydropurin-2,6-dion MS: M+1 = 426

### Beispiel 18

8-(2-Amino-ethoxy)-7-but-2-ynyl-3-methyl-1-(2-oxo-2-phenyl-ethyl)-3,7-dihydro-purin-2,6-dion MS: M+1 = 396

### Beispiel 19

1(S)-2(R)-8-(1-Amino-indan-2-yloxy)-3-methyl-7-(3-methyl-but-2-enyl)-1-(2-oxo-2-phenylethyl)-3,7- dihydro-purin-2,6-dion-trifluoracetat MS: M+1 = 500

### Beispiel 20

8-(2-Amino-3-phenyl-propoxy)-3-methyl-7-(3-methyl-but-2-enyl)-1-(2-oxo-2-phenyl-ethyl)-3,7-dihydro-purin-2,6-dion-trifluoracetat MS: M+1 = 502

### Beispiel 21

1(R)-2(S)-8-(1-Amino-indan-2-yloxy)-3-methyl-7-(3-methyl-but-2-enyl)-1-(2-oxo-2-phenylethyl)-3,7- dihydro-purin-2,6-dion-trifluoracetat MS: M+1 = 500

### Beispiel 22

2-[8-(2-Amino-ethoxy)-1,3-dimethyl-2,6-dioxo-1,2,3,6-tetrahydro-purin-7-ylmethyl]-benzonitril MS: M+1 = 455

### Beispiel 23

8-(2-Amino-ethoxy)-1,3-dimethyl-7-(3-phenyl-allyl)-3,7-dihydro-purin-2,6-dion MS: M+1 = 455

### Beispiel 24

8-(2-Amino-ethoxy)-7-(3-methyl-but-2-enyl)-1-(2-oxo-2-phenyl-ethyl)-3-(2,2,2-trifluoroethyl)-3,7-dihydro-purin-2,6-dion-hydrochlorid MS: M+1 = 480

### Beispiel 25

R-3-Methyl-7-(3-methyl-but-2-enyl)-1-(2-oxo-2-phenyl-ethyl)-8-(pyrrolidin-2-ylmethoxy)-3,7-dihydro-purin-2,6-dion MS: M+1 = 452

### Beispiel 26

R-3-Methyl-7-(3-methyl-but-2-enyl)-1-(2-oxo-2-phenyl-ethyl)-8-(pyrrolidin-3-yloxy)-3,7-dihydro-purine-2,6-dion MS: M+1 = 438

### Beispiel 27

S-3-Methyl-7-(3-methyl-but-2-enyl)-1-(2-oxo-2-phenyl-ethyl)-8-(pyrrolidin-3-yloxy)-3,7-dihydro-purine-2,6-dion MS: M+1 = 438

### Beispiel 28

Cis-8-(2-Aminomethyl-cyclohexyloxy)-3-methyl-7-(3-methyl-but-2-enyl)-1-(2-oxo-2-phenylethyl)- 3,7-dihydro-purin-2,6-dion MS: M+1 = 480

### Beispiel 29

Trans-8-(2-Amino-cyclohexylmethoxy)-3-methyl-7-(3-methyl-but-2-enyl)-1-(2-oxo-2-phenylethyl)- 3,7-dihydro-purin-2,6-dion MS: M+1 = 480

### Beispiel 30

Cis-8-(2-Amino-cyclohexylmethoxy)-3-methyl-7-(3-methyl-but-2-enyl)-1-(2-oxo-2-phenylethyl)- 3,7-dihydro-purin-2,6-dion MS: M+1 = 480

### Beispiel 31

Trans- 8-(2-Amino-cyclopentyloxy)-3-methyl-7-(3-methyl-but-2-enyl)-1-(2-oxo-2-phenylethyl)-3,7- dihydro-purin-2,6-dion MS: M+1 = 452

### Beispiel 32

Trans- 8-(2-Amino-cyclohexyloxy)-3-methyl-7-(3-methyl-but-2-enyl)-1-(2-oxo-2-phenylethyl)-3,7- dihydro-purin-2,6-dion-hydrochlorid MS: M+1 = 466

### Beispiel 33

8-(3-Amino-2,2-dimethyl-propoxy)-3-methyl-7-(3-methyl-but-2-enyl)-1-(2-oxo-2-phenylethyl)-3,7-dihydro-purin-2,6-dion-hydrochlorid MS: M+1 = 454

### Beispiel 34

3-Methyl-7-(3-methyl-but-2-enyl)-1-(2-oxo-2-phenyl-ethyl)-8-(2,2,6,6-tetramethyl- piperidin-4-yloxy)-3,7-dihydro-purin-2,6-dion MS: M+1 = 508

### Beispiel 35

8-(3-Amino-2-methyl-propoxy)-3-methyl-7-(3-methyl-but-2-enyl)-1-(2-oxo-2-phenyl-ethyl)-3,7-dihydro-purin-2,6-dion MS: M+1 = 440

### Beispiel 36

Trans-8-(2-Aminomethyl-cyclohexyloxy)-3-methyl-7-(3-methyl-but-2-enyl)-1-(2-oxo-2-phenyl-ethyl)- 3,7-dihydro-purin-2,6-dion (Diastereomerengemisch) MS: M+1 = 480

### Beispiel 37

Trans-8-(2-Aminomethyl-cyclohexyloxy)-3-methyl-7-(3-methyl-but-2-enyl)-1-(2-oxo-2-phenyl-ethyl)- 3,7-dihydro-purin-2,6-dion-hydrochlorid (Diastereomerengemisch) MS: M+1 = 480

### Beispiel 38

Trans-8-(2-Aminomethyl-cyclohexyloxy)-3-methyl-7-(3-methyl-but-2-enyl)-1-(2-oxo-2-phenyl-ethyl)- 3,7-dihydro-purin-2,6-dion (Diastereomer 1)
Diese Verbindung wurde durch chromatografische Trennung des Diastereomerengemisches (Beispiel 36) an einer chiralen Säule (Chiralpak AD 10x40 cm, Hersteller Fa. Merck; Eluent: Methanol + 0,1% Diethylamin; Fluß:200 ml/min) als schneller eluierendes Isomeres erhalten. MS: M+1 = 480 Fp.: 151,7°C

### Beispiel 39

Trans-8-(2-Aminomethyl-cyclohexyloxy)-3-methyl-7-(3-methyl-but-2-enyl)-1-(2-oxo-2-phenyl-ethyl)- 3,7-dihydro-purin-2,6-dion (Diastereomer 2)
Diese Verbindung wurde durch chromatografische Trennung des Diastereomerengemisches (Beispiel 36) an einer chiralen Säule (Chiralpak AD 10x40 cm, Hersteller Fa. Merck; Eluent: Methanol + 0,1% Diethylamin; Fluß:200 ml/min) als Isomeres mit der längeren Retentionszeit erhalten.
MS: M+1 = 480 Fp.: zähes Harz

### Beispiel 40

Trans- 8-(2-Amino-cyclohexyloxy)-3-methyl-7-(3-methyl-but-2-enyl)-1-(2-oxo-2-phenylethyl)-3,7- dihydro-purin-2,6-dion-hydrochlorid (Diastereomer 1)
Diese Verbindung wurde durch chromatografische Trennung des Diastereomerengemisches (Beispiel 32) an einer chiralen Säule (Chiralpak AD 10x40 cm, Hersteller Fa. Merck; Eluent: Methanol + 0,1% Diethylamin; Fluß:200 ml/min) als schneller eluierendes Isomeres erhalten.
MS: M+1 = 466

### Beispiel 41

Trans- 8-(2-Amino-cyclohexyloxy)-3-methyl-7-(3-methyl-but-2-enyl)-1-(2-oxo-2-phenylethyl)-3,7- dihydro-purin-2,6-dion-hydrochlorid (Diastereomer 2)

Diese Verbindung wurde durch chromatografische Trennung des Diastereomerengemisches (Beispiel 32) an einer chiralen Säule (Chiralpak AD 10x40 cm, Hersteller Fa. Merck; Eluent: Methanol + 0,1% Diethylamin; Fluß:200 ml/min) als Isomeres mit der längeren Retentionszeit erhalten.
MS: M+1 = 466

### Beispiel 42

Trans-8-(2-Amino-cyclohexyloxy)-7-(2-chlor-benzyl)-1,3-dimethyl-3,7-dihydro-purin-2,6-dion MS: M+1 = 418

Die folgenden Beispiele erläutern die Herstellung der Ausgangsstoffe:

### Beispiel 43

8-Brom-3-methyl-3,7-dihydro-purin-2,6-dion
100g 3-Methyl-3,7-dihydro-purin-2,6-dion und 49g Natriumacetat werden in 800ml Eisessig suspendiert, auf 90°C Innentemperatur erhitzt und langsam (ca. 3-4Stunden) mit 33,3ml Brom versetzt. Die Suspension wird noch 3 Stunden bei dieser Temperatur nachgerührt, laut DC (DCM/MeOH 10:1) ist die Reaktion vollständig. Die Reaktionslösung wird abgekühlt und abgesaugt. Der Rückstand wird mit 100ml Eisessig und 500ml Wasser gewaschen und im Vakuum bei 50°C getrocknet.
Ausbeute: 145 g Fp.: >300°C (Zers.)

### Beispiel 44

8-Brom-3-methyl-7-(3-methyl-but-2-enyl)-3,7-dihydro-purin-2,6-dion
50g 8-Brom-3-methyl-3,7-dihydro-purin-2,6-dion werden in 900ml DMF vorgelegt und mit 14,1g Kaliumcarbonat versetzt. Die Suspension wird auf 60°C Innentemperatur erhitzt und langsam mit 31,7g 1-Brom-3-methyl-2-buten versetzt. Es wird noch für 3 Stunden bei dieser Temperatur nachgerührt und anschließend mit 1,2L Wasser versetzt. Der Feststoff wird abgesaugt, gut mit Wasser gewaschen und bei 45°C im Vakuum getrocknet.
Ausbeute: 63,9 g Fp.: 223,3°C

### Beispiel 45

8-Brom-3-methyl-7-(3-methyl-but-2-enyl)-1-(2-oxo-2-phenyl-ethyl)-3,7-dihydro-purin-2,6-dion
30 g 8-Brom-3-methyl-7-(3-methyl-but-2-enyl)-3,7-dihydro-purin-2,6-dion, 20,6 g Phenacylbromid und 25,4 g Pottasche werden in 400ml DMF vorgelegt und für 3 Stunden auf 80°C Innentemperatur erhitzt. Die Lösung wird auf ca. 50°C abgekühlt und langsam mit 400ml Wasser versetzt. Anschließend wird über Nacht gerührt und vom ausgefallenen Feststoff abgesaugt. Nach dem Waschen mit Wasser wird der hellbraune Feststoff aus 300ml Isopropanol umkristallisiert.
Ausbeute: 39,7 g Fp.: 90,4°C

## Patentansprüche

1. Verbindungen der Formel I, 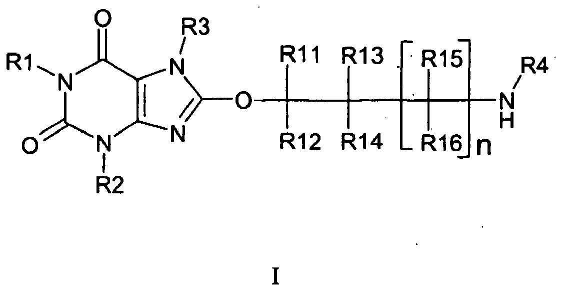 worin bedeuten
R1, R2, R3 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, NO₂, SF₅, OH, (C₁-C₆)-Alkyl, -CF₃, -OCF₃, -SCF₃, (C₂-C₆)-Alkenyl, (C₂-C₆)-Alkinyl, OR7, OP(O)(OR7)₂, NR7R8, NR7CONR7R8, COR7, OCOR7, OCOOR7, COOR7, CONR7R8, OCONR7R8, (C₁-C₆)-Alkylen-OR7, (C₁-C₆)-Alkylen-NR7R8, (C₁-C₆)-Alkylen-NR7SO₂R7, (C₁-C₆)-Alkylen-SR7, Alkylen-S(O)R7, Alkylen-CONR7R8, SR7, SOR7, SO₂R7, SO₂NR7R8, NR7SO₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl; wobei nicht alle Reste R1, R2 und R3 gleichzeitig Wasserstoff oder Methyl sein dürfen;
R7, R8 unabhängig voneinander H, (C₁-C₆)-Alkyl, (C₃-C₁₀)-Cycloalkyl, Aryl, Heterocyclyl, (C₁-C₄)-Alkylen-(C₆-C₁₀)-Aryl oder (C₁-C₄)-Alkylen-Heterocyclyl;
R4 Wasserstoff, (C₁-C₆)-Alkyl oder (C₃-C₈)-Cykloalkyl, wobei (C₁-C₆)-Alkyl oder (C₃-C₈)-Cykloalkyl substitiuert sein können mit F, Cl, Br, I, CN, Aryl, Heterocyclyl, NH₂, NH(C₁-C₆)-Alkyl, N((C₁-C₆)-Alkyl)₂, OH, O(C₁-C₆)-Alkyl, OAryl, OHeteroaryl, S(C₁-C₆)-Alkyl, S(O)(C₁-C₆)-Alkyl, S(O)₂(C₁-C₆)-Alkyl;
R11, R12, R13, R14, R15, R16 unabhängig voneinander H, (C₁-C₆)-Alkyl, (C₃-C₈)-Cykloalkyl, (C₁-C₄)-Alkylen-Aryl oder (C₁-C₄)-Alkylen-Heterocyclyl;
oder zwei der Reste R4, R11, R12, R13, R14, R15, R16 bilden gemeinsam einen (C₂-C₆)-Alkylenrest;
n 0;
wobei die Verbindung 1,3-Di(cyclopropylmethyl)-7-(4-methoxybenzyl-)-8-(4-piperidinyloxy)xanthine ausgenommen ist,
sowie deren physiologisch verträglichen Salze.

2. Verbindungen der Formel I, gemäß Anspruch 1, **dadurch gekennzeichnet, dass** darin bedeuten
R1, R2, R3 unabhängig voneinander (C₁-C₆)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, CN, NO₂, SF₅, OH, (C₁-C₆)-Alkyl, -CF₃, -OCF₃, - SCF₃, (C₂-C₆)-Alkenyl, (C₂-C₆)-Alkinyl, OR7, NR7R8, NR7CONR7R8, COR7, COOR7, CONR7R8, (C₁-C₆)-Alkylen-OR7, (C₁-C₆)-Alkylen-NR7R8, (C₁-C₆)-Alkylen-NR7SO₂R7, (C₁-C₆)-Alkylen-SR7, Alkylen-S(O)R7, Alkylen-CONR7R8, SR7, SOR7, SO₂R7, SO₂NR7R8, NR7SO₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl; wobei nicht alle Reste R1, R2 und R3 gleichzeitig Methyl sein dürfen;
R7, R8 unabhängig voneinander H, (C₁-C₆)-Alkyl, (C₃-C₁₀)-Cycloalkyl, Aryl, Heterocyclyl (C₁-C₄)-Alkylen-(C₆-C₁₀)-Aryl oder (C₁-C₄)-Alkylen-Heterocyclyl;
R4 Wasserstoff;
R11, R12, R13, R14 unabhängig voneinander H, (C₁-C₆)-Alkyl oder (C₃-C₈) Cycloalkyl, (C₁-C₄)-Alkylen-Aryl oder (C₁-C₄)-Alkylen-Heterocyclyl;
n 0;
sowie deren physiologisch verträglichen Salze.

3. Verbindungen der Formel I, gemäß Anspruch 1, **dadurch gekennzeichnet, dass** darin bedeuten
R1, R2, R3 unabhängig voneinander (C₁-C₆)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, CN, SF₅, (C₁-C₆)-Alkyl, -CF₃, -OCF₃, -SCF₃, (C₂-C₄)-Alkenyl, (C₂-C₄)-Alkinyl, OR7, NR7R8, NR7CONR7R8, COR7, COOR7, CONR7R8, (C₁-C₄)-Alkylen-OR7, (C₁-C₄)-Alkylen-NR7R8, (C₁-C4)-Alkylen-NR7SO₂R7, (C₁-C₆)-Alkylen-SR7, Alkylen-S(O)R7, Alkylen-CONR7R8, SR7, SOR7, SO₂R7, SO₂NR7R8, NR7SO₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl; wobei nicht alle Reste R1, R2 und R3 gleichzeitig Methyl sein dürfen;
R7, R8 unabhängig voneinander H, (C₁-C₆)-Alkyl, (C₃-C₁₀)-Cycloalkyl, Aryl, Heterocyclyl, (C₁-C₄)-Alkylen-(C₆-C₁₀)-Aryl oder (C₁-C₄)-Alkylen-Heterocyclyl;
R4 Wasserstoff;
R11, R12, R13, R14 unabhängig voneinander H, (C₁-C4)-Alkyl;
n 0;
sowie deren physiologisch verträglichen Salze.

4. Verbindungen gemäß einem oder mehreren der Ansprüche 1 bis 3 zur Anwendung als Arzneimittel.

5. Arzneimittel enthaltend eine oder mehrere der Verbindungen gemäß einem oder mehreren der Ansprüche 1 bis 3.

6. Arzneimittel enthaltend eine oder mehrere der Verbindungen gemäß einem oder mehreren der Ansprüche 1 bis 3 und mindestens einen weiteren Wirkstoff.

7. Arzneimittel, gemäß Anspruch 6, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alfa Agonisten, PPAR alfa/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

8. Verwendung der Verbindungen gemäß einem oder mehreren der Ansprüche 1 bis 3 zur Herstellung eines Medikamentes zur Blutzuckersenkung.

9. Verwendung der Verbindungen gemäß einem oder mehreren der Ansprüche 1 bis 3 zur Herstellung eines Medikamentes zur Behandlung des Typ II Diabetes.

10. Verwendung der Verbindungen gemäß einem oder mehreren der Ansprüche 1 bis 3 zur Herstellung eines Medikamentes zur Behandlung von Lipid- und Kohlenhydratstoffwechselstörungen.

11. Verwendung der Verbindungen gemäß einem oder mehreren der Ansprüche 1 bis 3 zur Herstellung eines Medikaments zur Behandlung arteriosklerotischer Erscheinungen.

12. Verwendung der Verbindungen gemäß einem oder mehreren der Ansprüche 1 bis 3 zur Herstellung eines Medikaments zur Behandlung von Insulin Resistenz.

13. Verfahren zur Herstellung eines Arzneimittels enthaltend eine oder mehrere der Verbindungen gemäß einem oder mehreren der Ansprüche 1 bis 3, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 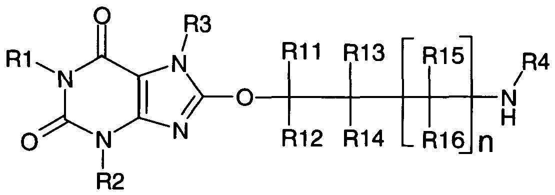 in which the meanings are
R1, R2, R3 independently of one another H, (C₁-C₁₀)-alkyl, (C₃-C₁₀) -cycloalkyl, (C₂-C₁₀)-alkenyl , (C₂-C₁₀) -alkynyl, (C₆-C₁₀) -aryl , heterocyclyl, where the alkyl, cycloalkyl, alkenyl, alkynyl, aryl and heterocyclyl radicals may be substituted one or more times by F, Cl, Br, I, CN, NO₂, SF₅, OH, (C₁-C₆) - alkyl, -CF₃ , -OCF₃ , -SCF₃ , (C₂-C₆)-alkenyl, (C₂-C₆)-alkynyl, OR7, OP(O)(OR7)₂, NR7R8, NR7CONR7R8, COR7, OCOR7, OCOOR7, COOR7, CONR7R8, OCONR7R8, (C₁-C₆)-alkylene-OR7, (C₁-C₆)-alkylene-NR7R8, (C₁-C₆)-alkylene-NR7SO₂R7, (C₁-C₆)-alkylene-SR7, alkylene-S(O)R7, alkylene-CONR7R8, SR7, SOR7, SO₂R7, SO₂NR7R8, NR7SO₂R7, (C₁-C₆) -alkylene- (C₃-C₁₀) -cycloalkyl, (C₁-C₆)-alkylene- (C₆-C₁₀)-aryl, (C₁-C₆) - alkylene-heterocyclyl, (C₃-C₁₀)-cycloalkyl, (C₆-C₁₀)-aryl or heterocyclyl; where not all radicals R1, R2 and R3 may simultaneously be hydrogen or methyl;
R7, R8 independently of one another H, (C₁-C₆)-alkyl, (C₃-C₁₀)-cycloalkyl, aryl, heterocyclyl, (C₁-C₄)-alkylene-(C₆-C₁₀)-aryl or (C₁-C₄)-alkylene-heterocyclyl;
R4 hydrogen, (C₁-C₆)-alkyl or (C₃-C₈)-cycloalkyl, where (C₁-C₆)-alkyl or (C₃-C₈)-cycloalkyl may be substituted by F, Cl, Br, I, CN, aryl, heterocyclyl, NH₂, NH(C₁-C₆)-alkyl, N((C₁-C₆)-alkyl)₂, OH, O(C₁-C₆-alkyl, Oaryl, Oheteroaryl, S (C₁-C₆)-alkyl , S (O) (C₁-C₆) -alkyl, S(O)₂(C₁-C₆)-alkyl;
R11, R12, R13, R14, R15, R16 independently of one another H, (C₁-C₆)-alkyl , (C₃-C₈)-cycloalkyl, (C₁-C₄)-alkylene-aryl or (C₁-C₄)-alkylene-heterocyclyl;
or two of the radicals R4, R11, R12, R13, R14, R15, R16 together form a (C₂-C₆)-alkylene radical;
n 0;
where the compound 1,3-di(cyclopropylmethyl)-7-(4-methoxybenzyl)-8-(4-piperidinyloxy)xanthines is excluded,
and the physiologically tolerated salts thereof.

2. A compound of the formula I as claimed in claim 1, wherein the meanings are
R1, R2, R3 independently of one another (C₁-C₆)-alkyl, (C₃-C₁₀) -cycloalkyl, (C₂-C₁₀) -alkenyl, (C₂-C₁₀)-alkynyl, (C₆-C₁₀)-aryl, heterocyclyl, where the alkyl, cycloalkyl, alkenyl, alkynyl, aryl and heterocyclyl radicals may be substituted one or more times by F, Cl, Br, CN, NO₂, SF₅, OH, (C₁-C₆)-alkyl, -CF₃, -OCF₃, -SCF₃, (C₂-C₆)-alkenyl, (C₂-C₆)-alkynyl, OR7, NR7R8, NR7CONR7R8, COR7, COOR7, CONR7R8, (C₁-C₆)-alkylene-OR7, (C₁-C₆)-alkylene-NR7R8, (C₁-C₆)-alkylene-NR7SO₂R7, (C₁-C₆)-alkylene-SR7, alkylene-S(O)R7, alkylene-CONR7R8, SR7, SOR7, SO₂R7, SO₂NR7R8 , NR7SO₂R7 , (C₁-C₆)-alkylene-(C₃-C₁₀) -cycloalkyl, (C₁-C₆) -alkylene- (C₆-C₁₀) -aryl, (C₁-C₆)-alkylene-heterocyclyl, (C₃-C₁₀)-cycloalkyl, (C₆-C₁₀)-aryl or heterocyclyl; where not all radicals R1, R2 and R3 may simultaneously be methyl;
R7, R8 independently of one another H, (C₁-C₆)-alkyl , (C₃-C₁₀)-cycloalkyl, aryl, heterocyclyl (C₁-C₄)-alkylene-(C₆-C₁₀)-aryl or (C₁-C₄)-alkylene-heterocyclyl;
R4 hydrogen;
R11, R12, R13, R14 independently of one another H, (C₁-C₆)-alkyl or (C₃-C₈)-cycloalkyl, (C₁-C₄)-alkylene-aryl or (C₁-C₄)-alkylene-heterocyclyl;
n 0;
and the physiologically tolerated salts thereof.

3. A compound of the formula I as claimed in claim 1, wherein the meanings are
R1, R2, R3 independently of one another (C₁-C₆)-alkyl, (C₃-C₁₀)-cycloalkyl, (C₂-C₁₀)-alkenyl, (C₂-C₁₀)-alkynyl, (C₆-C₁₀)-aryl, heterocyclyl, where the alkyl, cycloalkyl, alkenyl, alkynyl, aryl and heterocyclyl radicals may be substituted one or more times by F, Cl, CN, SF₅, (C₁-C₆)-alkyl , -CF₃, -OCF₃ , -SCF₃, (C₂-C4)-alkenyl, (C₂-C4)-alkynyl , OR7, NR7R8, NR7CONR7R8, COR7, COOR7, CONR7R8, (C₁-C4)-alkylene-OR7, (C₁-C4)-alkylene-NR7R8, (C₁-C4)-alkylene-NR7SO₂R7, (C₁-C₆)-alkylene-SR7, alkylene-S(O)R7, alkylene-CONR7R8, SR7, SOR7, SO₂R7, SO₂NR7R8, NR7SO₂R7, (C₁-C₆)-alkylene- (C₃-C₁₀)-cycloalkyl, (C₁-C₆) -alkylene-(C₆-C₁₀)-aryl, (C₁-C₆) -alkylene-heterocyclyl , (C₃-C₁₀) -cycloalkyl, (C₆-C₁₀) -aryl or heterocyclyl; where not all radicals R1, R2 and R3 may simultaneously be methyl;
R7, R8 independently of one another H, (C₁-C₆)-alkyl, (C₃-C₁₀)-cycloalkyl, aryl, heterocyclyl, (C₁-C₄)-alkylene-(C₆-C₁₀)-aryl or (C₁-C₄)-alkylene-heterocyclyl;
R4 hydrogen;
R11, R12, R13, R14 independently of one another H, (C₁-C4)-alkyl;
n 0;
and the physiologically tolerated salts thereof.

4. A compound as claimed in one or more of claims 1 to 3 for use as medicament.

5. A medicament comprising one or more of the compounds as claimed in one or more of claims 1 to 3.

6. A medicament comprising one or more of the compounds as claimed in one or more of claims 1 to 3 and at least one other active ingredient.

7. The medicament as claimed in claim 6, which comprises as other active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed sertoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

8. The use of the compounds as claimed in one or more of claims 1 to 3 for producing a medicament for lowering blood glucose.

9. The use of the compounds as claimed in one or more of claims 1 to 3 for producing a medicament for the treatment of type II diabetes.

10. The use of the compounds as claimed in one or more of claims 1 to 3 for producing a medicament for the treatment of disturbances of lipid and carbohydrate metabolism.

11. The use of the compounds as claimed in one or more of claims 1 to 3 for producing a medicament for the treatment of arteriosclerotic manifestations.

12. The use of the compounds as claimed in one or more of claims 1 to 3 for producing a medicament for the treatment of insulin resistance.

13. A process for producing a medicament comprising one or more of the compounds as claimed in one or more of claims 1 to 3, wherein the active ingredient is mixed with a pharmaceutically suitable carrier and this mixture is converted into a form suitable for administration.

## Revendications

1. Composés de formule I 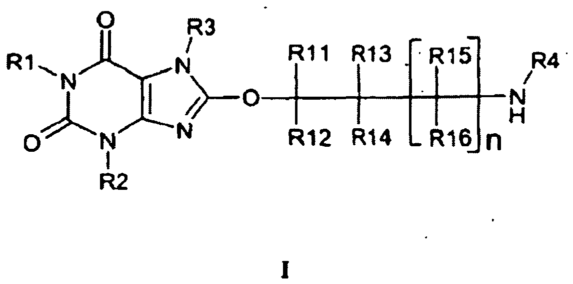 dans laquelle
R1, R2, R3 signifient indépendamment les uns des autres H, alkyle en (C₁-C₁₀) , cycloalkyle en (C₃-C₁₀) , alcényle en (C₂-C₁₀), alcynyle en (C₂-C₁₀), aryle en (C₆-C₁₀), hétérocyclyle, les radicaux alkyle, cycloalkyle, alcényle, alcynyle, aryle et hétérocyclyle pouvant être substitués une ou plusieurs fois avec F, Cl, Br, I, CN, NO₂, SF₅, OH, alkyle en (C₁-C₆) , -CF₃, -OCF₃, -SCF₃, alcényle en (C₂-C₆) alcynyle en (C₂-C₆), OR7, OP(O)(OR7)₂, NR7R8, NR7CONR7R8, COR7, OCOR7, OCOOR7, COOR7, CONR7R8, OCONR7R8, alkylène en (C₁-C₆) -OR7, alkylène en (C₁-C₆)-NR7R8, alkylène en (C₁-C₆)-NR7SO₂R7, alkylène en (C₁-C₆)-SR7, alkylène-S(O)R7, alkylène-CONR7R8, SR7, SOR7, SO₂R7, SO₂NR7R8, NR7SO₂R7, alkylène en (C₁-C₆)-cycloalkyle en (C₃-C₁₀), alkylène en (C₁-C₆) - aryle en (C₆-C₁₀), alkylène en (C₁-C₆)-hétérocyclyle, cycloalkyle en (C₃-C₁₀), aryle en (C₆-C₁₀) ou hétérocyclyle ; tous les radicaux R1, R2 et R3 ne pouvant pas représenter simultanément hydrogène ou méthyle ;
R7, R8 signifient indépendamment l'un de l'autre H, alkyle en (C₁-C₆), cycloalkyle en (C₃-C₁₀), aryle, hétérocyclyle, alkylène en (C₁-C₄)-aryle en (C₆-C₁₀) ou alkylène en (C₁-C₄)-hétérocyclyle ;
R4 signifie hydrogène, alkyle en (C₁-C₆) ou cycloalkyle en (C₃-C₈), alkyle en (C₁-C₆) ou cycloalkyle en (C₃-C₈) pouvant être substitué avec F, Cl, Br, I, CN, aryle, hétérocyclyle, NH₂, NH-alkyle en (C₁-C₆), N(alkyle en (C₁-C₆))₂, OH, O-alkyle en (C₁-C₆), O-aryle, O-hétéroaryle, S-alkyle en (C₁-C₆), S(O)-alkyle en (C₁-C₆), S(O)₂-alkyle en (C₁-C₆) ;
R11, R12, R13, R14, R15, R16 signifient indépendamment les uns des autres H, alkyle en (C₁-C₆), cycloalkyle en (C₃-C₈), alkylène en (C₁-C₄)-aryle ou alkylène en (C₁-C₄)-hétérocyclyle ;
ou deux des radicaux R4, R11, R12, R13, R14, R15, R16 forment ensemble un radical alkylène en (C₂-C₆) ;
n signifie 0 ;
le composé 1,3-di(cyclopropylméthyl)-7-(4-méthoxybenzyl)-8-(4-pipéridinyloxy)xanthine étant exclu,
ainsi que leurs sels physiologiquement compatibles.

2. Composés de formule I selon la revendication 1, **caractérisés en ce que**, dans celle-ci,
R1, R2, R3 signifient indépendamment les uns des autres alkyle en (C₁-C₆), cycloalkyle en (C₃-C₁₀), alcényle en (C₂-C₁₀), alcynyle en (C₂-C₁₀), aryle en (C₆-C₁₀), hétérocyclyle, les radicaux alkyle, cycloalkyle, alcényle, alcynyle, aryle et hétérocyclyle pouvant être substitués une ou plusieurs fois avec F, Cl, Br, CN, NO₂, SF₅, OH, alkyle en (C₁-C₆), -CF₃, -OCF₃, -SCF₃, alcényle en (C₂-C₆), alcynyle en (C₂-C₆), OR7, NR7R8, NR7CONR7R8, COR7, COOR7, CONR7R8, alkylène en (C₁-C₆)-OR7, alkylène en (C₁-C₆) -NR7R8, alkylène en (C₁-C₆)-NR7SO₂R7, alkylène en (C₁-C₆) -SR7, alkylène-S(O)R7, alkylène-CONR7R8, SR7, SOR7, SO₂R7, SO₂NR7R8, NR7SO₂R7, alkylène en (C₁-C₆)-cycloalkyle en (C₃-C₁₀), alkylène en (C₁-C₆) -aryle en (C₆-C₁₀) , alkylène en (C₁-C₆)-hétérocyclyle, cycloalkyle en (C₃-C₁₀), aryle en (C₆-C₁₀) ou hétérocyclyle ; tous les radicaux R1, R2 et R3 ne pouvant pas représenter simultanément méthyle ;
R7, R8 signifient indépendamment l'un de l'autre H, alkyle en (C₁-C₆), cycloalkyle en (C₃-C₁₀), aryle, hétérocyclyle, alkylène en (C₁-C₄)-aryle en (C₆-C₁₀) ou alkylène en (C₁-C₄)-hétérocyclyle ;
R4 signifie hydrogène ;
R11, R12, R13, R14 signifient indépendamment les uns des autres H, alkyle en (C₁-C₆) ou cycloalkyle en (C₃-C₈) , alkylène en (C₁-C₄)-aryle ou alkylène en (C₁-C₄) - hétérocyclyle ;
n signifie 0 ;
ainsi que leurs sels physiologiquement compatibles.

3. Composés de formule I selon la revendication 1, **caractérisés en ce que**, dans celle-ci,
R1, R2, R3 signifient indépendamment les uns des autres alkyle en (C₁-C₆), cycloalkyle en (C₃-C₁₀), alcényle en (C₂-C₁₀), alcynyle en (C₂-C₁₀) , aryle en (C₆-C₁₀), hétérocyclyle, les radicaux alkyle, cycloalkyle, alcényle, alcynyle, aryle et hétérocyclyle pouvant être substitués une ou plusieurs fois avec F, Cl, CN, SF₅, alkyle en (C₁-C₆) , -CF₃, -OCF₃, -SCF₃, alcényle en (C₂-C₄), alcynyle en (C₂-C₄), OR7, NR7R8, NR7CONR7R8 , COR7, COOR7, CONR7R8, alkylène en (C₁-C₄)-OR7, alkylène en (C₁-C₄)-NR7R8, alkylène en (C₁-C₄) -NR7SO₂R7, alkylène en (C₁-C₆)-SR7, alkylène-S(O)R7, alkylène-CONR7R8, SR7, SOR7, SO₂R7, SO₂NR7R8 , NR7SO₂R7 , alkylène en (C₁-C₆)-cycloalkyle en (C₃-C₁₀), alkylène en (C₁-C₆)-aryle en (C₆-C₁₀), alkylène en (C₁-C₆)-hétérocyclyle, cycloalkyle en (C₃-C₁₀), aryle en (C₆-C₁₀) ou hétérocyclyle ; tous les radicaux R1, R2 et R3 ne pouvant pas représenter simultanément méthyle ;
R7, R8 signifient indépendamment l'un de l'autre H, alkyle en (C₁-C₆), cycloalkyle en (C₃-C₁₀), aryle, hétérocyclyle, alkylène en (C₁-C₄)-aryle en (C₆-C₁₀) ou alkylène en (C₁-C₄)-hétérocyclyle ;
R4 signifie hydrogène ;
R11, R12, R13, R14 signifient indépendamment les uns des autres H, alkyle en (C₁-C₄) ;
n signifie 0 ;
ainsi que leurs sels physiologiquement compatibles.

4. Composés selon une ou plusieurs des revendications 1 à 3 pour une utilisation en tant que médicament.

5. Médicament contenant un ou plusieurs des composés selon une ou plusieurs des revendications 1 à 3.

6. Médicament contenant un ou plusieurs des composés selon une ou plusieurs des revendications 1 à 3 et au moins un agent actif supplémentaire.

7. Médicament selon la revendication 6, **caractérisé en ce qu'**il contient en tant qu'agent actif supplémentaire un ou plusieurs antidiabétiques, agents actifs hypoglycémiques, inhibiteurs d'HMGCoA-réductase, inhibiteurs de résorption du cholestérol, agonistes de PPAR gamma, agonistes de PPAR alpha, agonistes de PPAR alpha/gamma, fibrates, inhibiteurs de MTP, inhibiteurs de résorption d'acides biliaires, inhibiteurs de CETP, adsorbeurs polymères d'acides biliaires, inducteurs des récepteurs de LDL, inhibiteurs d'ACAT, antioxydants, inhibiteurs de lipoprotéine-lipase, inhibiteurs d'ATP-citrate-lyase, inhibiteurs de squalène synthétase, antagonistes de lipoprotéine (a), inhibiteurs de lipase, insulines, sulfonylurées, biguanides, méglitinides, thiazolidinediones, inhibiteurs d'α-glucosidase, agents actifs agissant sur le canal calcique des cellules bêta dépendant de l'ATP, agonistes de CART, agonistes de NPY, agonistes de MC4, agonistes d'orexine, agonistes d'H3, agonistes de TNF, agonistes de CRF, antagonistes de CRF BP, agonistes d'urocortine, agonistes de β3, antagonistes du récepteur de CB1, agonistes de MSH (hormone stimulant les mélanocytes), agonistes de CCK, inhibiteurs du recaptage de sérotonine, composés de sertonine et noradrénergiques mixtes, agonistes de 5HT, agonistes de bombésine, antagonistes de galanine, hormones de croissance, composés libérant des hormones de croissance, agonistes de TRH, modulateurs de protéine découplante 2 ou 3, agonistes de leptine, agonistes de DA (bromocriptine, dopréxine), inhibiteurs de lipase/amylase, modulateurs de PPAR, modulateurs de RXR ou agonistes de TR-β ou amphétamines.

8. Utilisation des composés selon une ou plusieurs des revendications 1 à 3 pour la fabrication d'un médicament pour la diminution de la glycémie.

9. Utilisation des composés selon une ou plusieurs des revendications 1 à 3 pour la fabrication d'un médicament pour le traitement du diabète de type II.

10. Utilisation des composés selon une ou plusieurs des revendications 1 à 3 pour la fabrication d'un médicament pour le traitement des troubles du métabolisme des lipides et des glucides.

11. Utilisation des composés selon une ou plusieurs des revendications 1 à 3 pour la fabrication d'un médicament pour le traitement des phénomènes artériosclérotiques.

12. Utilisation des composés selon une ou plusieurs des revendications 1 à 3 pour la fabrication d'un médicament pour le traitement de la résistance à l'insuline.

13. Procédé de fabrication d'un médicament contenant un ou plusieurs des composés selon une ou plusieurs des revendications 1 à 3, **caractérisé en ce que** l'agent actif est mélangé avec un véhicule pharmaceutiquement approprié et ce mélange est mis sous une forme appropriée pour l'administration.
